# EUROPEAN PATENT SPECIFICATION

(11) **EP 4 295 829 B1**
(45) Date of publication and mention of the grant of the patent: **25.03.2026**
(21) Application number: 23197013.8
(22) Date of filing: 07.05.2020
(51) Int. Cl.: A61F 13/42, A61F 13/84

(54) **DETECTION DEVICE FOR SMART ABSORBENT ARTICLES**
DETEKTIONSVORRICHTUNG FÜR INTELLIGENTE ABSORBIERENDE ARTIKEL
DISPOSITIF DE DÉTECTION POUR ARTICLES ABSORBANTS INTELLIGENTS

(43) Date of publication of application: 27.12.2023
(62) Divisional of application: 20173437.3
(73) Proprietor: Ontex BV, 9255 Buggenhout (BE); Ontex Group NV, 9320 Erembodegem (BE)
(72) Inventor: WEBER, Ainas, 53474 Bad Neuenahr-Ahrweiler (DE); HELLMOLD, Jens, 59269 Beckum (DE); HEIRMAN, Lisa, 9250 Waasmunster (BE); RONSMANS, Joris, 3001 Heverlee (BE)
(74) Representative: Macchetta, Andrea

(56) References cited:
- EP-A1- 3 260 096
- US-A1- 2019 046 364
- US-B2- 7 049 969
- US-B2- 8 299 317
- US-B2- 9 380 977

## Description

### TECHNICAL FIELD

The disclosure relates to detection devices suitable for use in absorbent articles such as baby or adult diapers, pants, or incontinence briefs for adults and/or children. Typically said absorbent articles comprising added electronics adapted to monitor voiding events such as exudate gushes within said absorbent articles. The disclosure further relates to said absorbent articles comprising said detection devices.

### BACKGROUND

Absorbent articles for personal hygiene are designed to absorb and contain bodily exudates, such as a large quantity of urine. Non-limiting examples of disposable absorbent articles include diapers, pants, training pants, pads, adult incontinence products, and feminine hygiene products (including, for example, sanitary napkins and tampons). Other examples of disposable absorbent articles include bandages and wound dressings. In some embodiments, for example, an absorbent article comprises several layers providing different functions, for example a topsheet, a backsheet and in-between an absorbent core, among other layers.

The function of the absorbent core is to absorb and retain the exudates for a prolonged amount of time, for example overnight for a diaper, minimize re-wet to keep the wearer dry and avoid soiling of clothes or bed sheets. The majority of currently marketed absorbent articles comprise as absorbent material a blend of comminuted wood pulp with superabsorbent polymers (SAP) in particulate form, also called absorbent gelling materials (AGM), see for example U.S. Pat. No. 5,151,092 (Buell). Absorbent articles having a core consisting essentially of SAP as absorbent material (so called "airfelt-free" cores) have also been proposed but are less common than traditional mixed cores (see e.g. WO2008/155699 and WO2012/052172).

A number of disclosures exist (see for example EP2496197B1, EP2739254B1, and EP2582341B1) directed to sensors to sense a condition such as temperature from body or moisture from incontinence. The sensor comprises a signal processing unit, a transmitter and a power supply, typically in form of a battery. These elements are arranged on a flexible substrate in low profile enabling disposition adjacent to the human body. A complex series of mathematical and statistical manipulations are then needed in order to determine wetness events and wetness levels.

While such devices allow monitoring conditions of the human body and can also be used as a moisture sensor, it represents also relatively costly solution. It would not be seen appropriate to dispose of the sensor together with a (disposable) absorbent article. If the sensor, however, is to be reused, the sensing area has potentially been exposed to moisture. Therefore this concept does not allow for simple usage.

Examples of articles that provide improvements to the above drawbacks are disclosed in EP3415130, WO/2018/228822, WO/2018/229017, EP3461257, and EP3451988. In all such disclosures, electronic components are printed on the backsheet of the absorbent article and various pocket arrangements are described to connect the detection device or clip-on unit to the absorbent article. Nevertheless, they fail to describe a unit especially designed for seamlessly securing itself to the absorbent article in such a way to limit dislodging thereof. Moreover, they fail to describe means that allow to control the position of a unit versus electronic components of the absorbent article in an effective and user-friendly manner.

US20190046364 discloses a notification system that provides notification of patient events such as movement and/or incontinence. The notification system provides for a plurality of different pressure sensor pads as well as an incontinence pad to be used in association with a single monitor.

A need exists for an inexpensive and/or more simple and effective detection means that can be joined to absorbent articles whilst allowing easy reading of information from, for example, a screen thereof and limiting accidental dislodging thereof during wearer's movements. Moreover, a need exists for a design that is compact and easy to insert and be retained into a pocket formed on the absorbent article on a controllable position within such a pocket.

### SUMMARY

The invention is as defined in the claims.

In a first aspect, the disclosure relates to a detection device comprising: a housing comprising a front face, a back face, and side edges connecting the front and back faces and disposed therebetween; and a clip element rotatably connected to the housing, wherein the clip element comprises one or more locking elements arranged to mechanically engage with at least a portion of the side edge when in a closed position, and wherein said clip element can be rotated from an open position, where the locking elements do not mechanically engage with said side edge, to said closed position, and wherein said detection device is suitable for coupling to an absorbent article and arranged such that when in said closed position at least one layer of said absorbent article is interposed between said housing and said clip element.

In a second aspect, the disclosure relates to absorbent article comprising the detection device herein described.

In a third aspect, the disclosure relates to a kit of parts comprising a plurality of absorbent articles and from 1 to less than 5, preferably less than 4, more preferably less than 3, detection devices.

In a fourth aspect, the disclosure relates to an array comprising a plurality of absorbent articles, wherein said array comprises absorbent articles having at least two different sizes and wherein a plurality of absorbent articles of at least one of the at least two different sizes further comprise an absorbent core comprising one or more channels substantially free of absorbent material, preferably wherein the absorbent core comprises one or more core wrap layers enclosing absorbent material therein and wherein a top layer of said core wrap is joined to a bottom layer of said core wrap to form said channels substantially free of absorbent material, preferably said channels being circumscribed by absorbent material.

### BRIEF DESCRIPTION OF THE FIGURES

**Fig. 1** depicts an absorbent article and detection device according to an embodiment herein.
**Fig. 2** depicts schematically a portion of an absorbent article and pocket according to an embodiment herein.
**Fig. 3** depicts a detection device according to an embodiment herein.
**Fig. 4** depicts a detection device according to an embodiment herein.
**Fig. 5** illustrates a hinge element according to an embodiment herein.
**Fig. 6** illustrates housing shapes according to embodiments herein.
**Fig. 7** illustrates detection devices according to embodiments herein.

### DETAILED DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1 % or less of and from the specified value, in so far such variations are appropriate to perform in the disclosed disclosure. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The expression "% by weight" (weight percent), here and throughout the description unless otherwise defined, refers to the relative weight of the respective component based on the overall weight of the formulation.

The recitation of numerical ranges by endpoints includes all numbers and fractions subsumed within that range, as well as the recited endpoints unless otherwise stated.

"Bonded" refers to the joining, adhering, connecting, attaching, or the like, of at least two elements. Two elements will be considered to be bonded together when they are bonded directly to one another or indirectly to one another, such as when each is directly bonded to intermediate elements.

The term "consisting essentially of" does not exclude the presence of additional materials which do not significantly affect the desired characteristics of a given composition or product. Exemplary materials of this sort would include, without limitation, pigments, antioxidants, stabilizers, surfactants, waxes, flow promoters, solvents, particulates and materials added to enhance processability of the composition.

The term "disposable" is used herein to describe absorbent articles that generally are not intended to be laundered or otherwise restored or reused as an absorbent article (i.e., they are intended to be discarded after a single use and, preferably, to be recycled, composted or otherwise disposed of in an environmentally compatible manner).

"Join", "joining", "joined", or variations thereof, when used in describing the relationship between two or more elements, means that the elements can be connected together in any suitable manner, such as by heat sealing, ultrasonic bonding, thermal bonding, by adhesives, stitching, or the like. Further, the elements can be joined directly together, or may have one or more elements interposed between them, all of which are connected together.

As used herein, the "body-facing" or "bodyside" or "skin-facing" surface means that surface of the article or component which is intended to be disposed toward or placed adjacent to the body of the wearer during ordinary use, while the "outward", "outward-facing", "garment-facing" or "garment-side" surface is on the opposite side, and is intended to be disposed to face away from the wearer's body during ordinary use. Such outward surface may be arranged to face toward or placed adjacent to the wearer's undergarments when the absorbent article is worn.

Embodiments of the articles and processes according to the disclosure will now be described. It is understood that technical features described in one or more embodiments maybe combined with one or more other embodiments without departing from the intention of the disclosure and without generalization therefrom.

### THE ABSORBENT ARTICLE

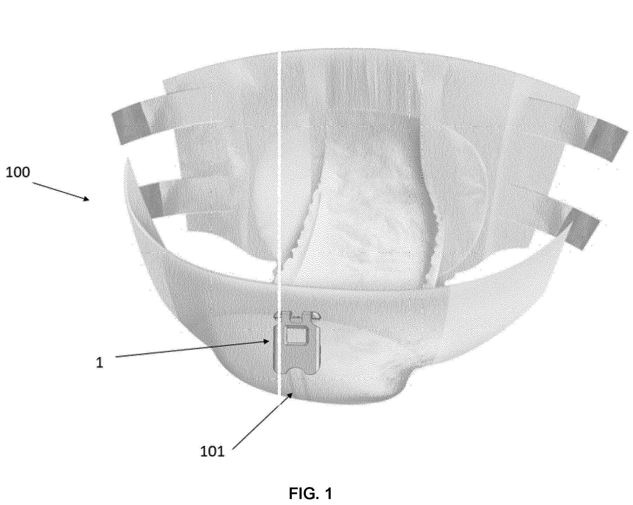

In an embodiment, the absorbent article (100) (as exemplified in Fig. 1) is typically a disposable diaper, pad or pant, and suitable for detecting an exudate and/or wetness (i.e. urine and/or feces) event therein and/or risk of exudate leakage therefrom, said absorbent article comprising: a liquid impermeable backsheet; a liquid permeable topsheet; and an absorbent core interposed between said backsheet and topsheet, wherein said backsheet comprises an exudate monitoring sensor positioned on a body facing surface thereof, preferably said exudate monitoring sensor comprising one or more conductive sensor tracks (101) typically comprising a conductive ink preferably printed onto said body facing surface of the liquid impermeable backsheet.

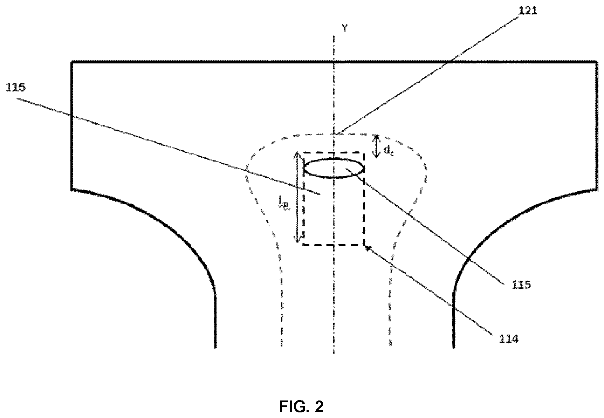

As exemplified in Fig.2, the absorbent article may comprise a pocket (116) comprising an opening (115) and a sealed perimeter (114) wherein the body facing surface of the liquid impermeable backsheet is joined to an insulating strip positioned between the absorbent core and said backsheet (typically such that the sensor tracks (101) are interposed between the body facing surface of the backsheet and a garment facing surface of the insulating strip, and arranged such that only said opening (115) is in fluid, typically liquid, communication with the rest of said pocket (116), typically said pocket (116) adapted for retaining therein at least a portion of the housing (2) of detection devices described herein may be positioned within a core region, and preferably at a distance **d**_{c} from a core proximal edge (121), said pocket (116) having a pocket length **L**ₚ, and wherein **d**_{c} is from 0.01**L**ₚ to 0.3**L**ₚ, preferably from 0.05**L**ₚ to 0.25**L**ₚ, more preferably from 0.08**L**ₚ to 0.20**L**ₚ. Advantageously such arrangement allows for good comfort and cushioning provided by the core layer through its thickness. By "within a core region" it is meant that the pocket is positioned such that a portion of the core is interposed between the subject and said pocket (and e.g. module when inserted/connected thereto) when the article is being worn.

The article may further comprise a removable detection device (or clip-on data processing module) (1) having a plurality of exposed electrically conductive terminals (8,8') capable of directly contacting the exudate monitoring sensor so as to be in electrical communication therewith.

In an embodiment, the position for the pocket (i.e. position of detection device attachment) is preferably chosen to ensure good wearing comfort and good accessibility for care givers. The placement is preferably at the front side of the wearer, below a belly button position, so that medical injections to the patient at this point are not hindered. The placement is also not too low and preferably above a pubic bone position to ensure good wearing comfort. The point of attachment is preferably at a position that provides some cushioning and buffering with the absorbent core underneath.

In an embodiment, the absorbent article comprises a liquid permeable topsheet, a liquid impermeable backsheet comprising an opening (115) for the detection device (1) to be inserted therein, and an absorbent core positioned between said topsheet and backsheet, wherein an insulating strip is positioned between said backseet and said absorbent core forming a pocket (116) adapted to retain the housing (2) of said detection device (1) therein. In a preferred embodiment, the opening (115) has an opening perimeter and the housing (2) has an in insertion perimeter, wherein the ratio of the opening perimeter and the insertion perimeter is from 0.9 to 1.3, preferably from 1.0 to 1.2. Preferably wherein the backsheet is arranged to deform (e.g. stretch) upon insertion of the housing (2) through the opening (115). Advantageously this allows to provide a tight fit between the portion of the housing (2) that is inserted into the pocket and the opening which mechanically inhibits dislodging and/or twisting of the device whilst inserting such that it is securely fitted. The absorbent article may further comprise any of the embodiments of the detection device described herein.

### THE DETECTION DEVICE

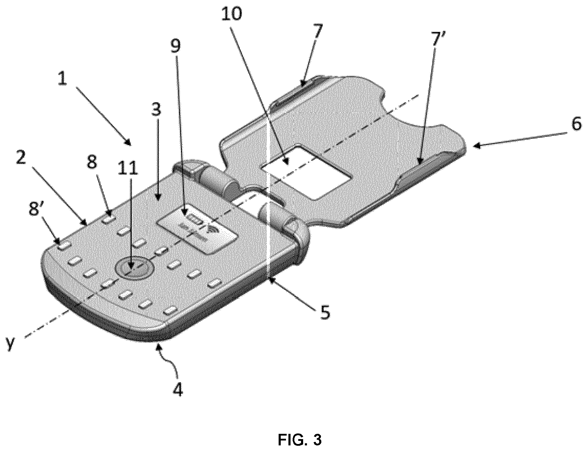

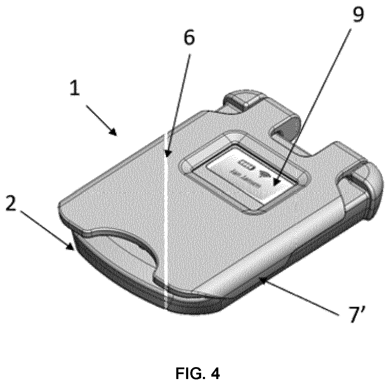

As exemplified in Figs. 3-7, the detection devices (1) herein comprise: a housing (2) comprising a front face (3), a back face (4), and side edges (5) connecting the front and back faces (3,4) and disposed therebetween; and a clip element (6) rotatably connected to the housing (2), wherein the clip element (6) comprises one or more locking elements (7,7') arrange to mechanically engage with at least a portion of the side edge (5) (preferably only a portion of said side edge typically said portion extending up to about half of the total depth of said side edge), when in a closed position, and wherein said clip element (6) can be rotated from an open position, where the locking elements (7,7') do not mechanically engage with said side edge (5), to said closed position, and wherein said detection device (1) is suitable for coupling to an absorbent article (100) and arranged such that when in said closed position at least one layer of said absorbent article (100) is interposed between said housing (2) and said clip element (6). Advantageously, this arrangement allows for a secure fit of the detection device to the absorbent article in an intuitive and cost-effective way without risking to damage and/or tear the backsheet layer that is interposed between the clip element and the housing when the device is connected to the absorbent article, the interposed layer being beneficial to strongly adhere the detection device to the absorbent article and ensure good electrical communication with the sensor tracks even when a subject moves around. It is undesirable that the locking elements of the clip element mechanically engage with a portion of the back face of the detection device as this could lead to hire chances of tearing.

In an embodiment, the housing (2) comprises therein a battery, a processor, and a transmitter, and further comprises a plurality of conductive terminals (8,8') that are exposed on the front face (3) of said housing (2) and facing said clip element (6) when in the closed position, said conductive terminals (8,8') being arranged to come into contact with one or more conductive sensor tracks (101) positioned on the absorbent article (100), preferably a surface, typically a body facing surface, of the at least one layer of said absorbent article interposed between the housing (2) and the clip element (6) when in the closed position, preferably said layer being a liquid impermeable backsheet of said absorbent article (100). Advantageously this allows for seamless and reliable connection with the conductive tracks of the absorbent article with limited risk of misalignment that would otherwise cause in exudate detection false measurements.

Preferably, the conductive terminals (8,8') are a plurality, preferably more than 4, more preferably more than 5, even more preferably from 6 to 20, and wherein said conductive terminals (8,8') are typically arranged in two or more rows along a length of the front face (3) of the housing (2) typically extending parallel to a longitudinal axis (y). This arrangement allows for a plurality of open circuits to be made and thus allow for multiple permutations of detection combinations when connected to the absorbent article that allows for improved flexibility of use. For example, the terminals may also be used to determine automatically at least one of the absorbency level, type, and/or size, of an absorbent article when connected thereto and to automatically communicate this information to a caregiver by sending a respective signal to a communication device such as a mobile device.

In an embodiment, the housing (2) further comprises a display (9) adapted to display information relating to a monitoring status, said display (9) being viewable from a garment facing side of the absorbent article (100) by a caregiver, said display (9) being positioned on the front face (3) of the housing (2). Advantageously by placing all electronics (including the screen on the housing, it allows for easy removal of the clip element for cleaning and/or replacement in case of damage without having to replace the electronic components hence resulting in a more cost-effective device in the longterm.

In an embodiment, the clip element (6) and the housing (2) further comprise a detachable hinge element arranged to permit single-handed removal of the clip element (6) from the housing (2). Typically being arranged such that it allows to easily disconnect the clip element from said housing, for example upon cleaning, and that allows to easily re-assemble said clip element to said housing again without tooling. Advantageously this prevents dirt traps that would result from a non-detachable mechanical hinge and/or it allows to use only one material type versus when a flexible material type would be selected for bending the clip versus the housing.

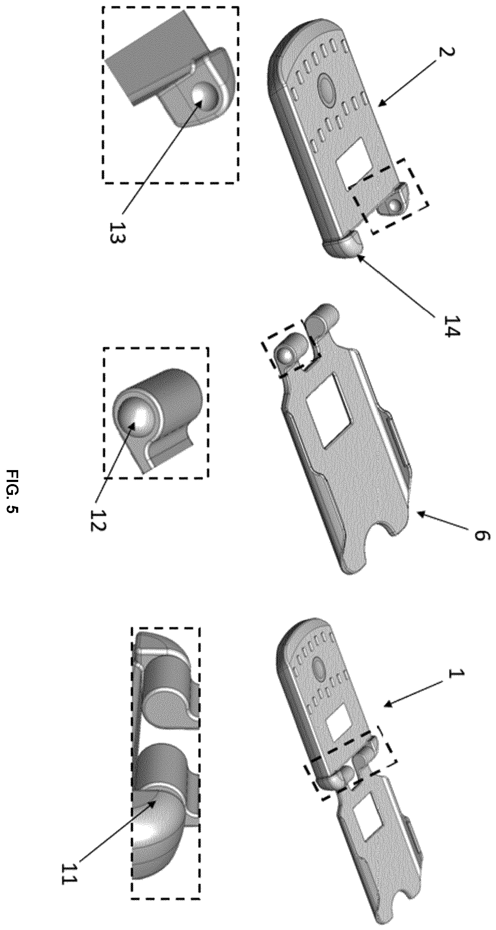

As exemplified in Fig. 5, the hinge element (11) may comprise a rounded protrusion (12) typically locate on the clip element (6) for mechanical engagement with a rounded recess (13) typically located on a shoulder (14) of the housing (2). Preferably, the hinge element consists of at least two oppositely disposed rounded protrusions (12) located on the clip element (6) and extending outwardly in opposite directions along an axis perpendicular to the longitudinal centerline y axis; and at least two oppositely disposed rounded recesses (13) located on opposing shoulders (14) of the housing (2). More preferably wherein each rounded protrusion engages each rounded recess such that said rounded protrusions are positioned inboard of said shoulders (14) of the housing (2) when the clip element (6) is joined to the housing (2). Advantageously this allows for easy and effective single-handed removal of the clip element for subsequent cleaning or replacement.

Preferably, the clip element (6) comprises a cut-out (10) or window sized such that the display (9) may protrude or be seen therethrough when in the closed position, preferably such that when in the closed position the display (9) can be viewed by a caregiver. This arrangement advantageously allows the display to be contained in the housing and the clip element to securely fasten thereto whilst not obstructing the visibility of the screen of the display.

Preferably, when attached to the absorbent article and in the closed position, the display (9) is exposed from the at least one layer of said absorbent article interposed between the housing (2) and the clip element (6); or the at least one layer is translucent such that information that is displayed onto the display (9) can be seen through said layer. It is advantageous for the layer, such as the backsheet of the absorbent article to be interposed between the housing and the clip element to provide a secure fit, this arrangement allows for a screen to be further viewed by a caregiver so that information may be displayed thereto such as relating to wetness and/or device status.

In an alternative embodiment, the housing (2) further comprises a display (9) adapted to display information relating to the detection device's status, said display (9) being viewable by a caregiver (for example to allow visual verification that the device indeed belongs to the intended wearer) before attaching the device to a diaper of a wearer and not viewable after it is attached. In this embodiment it is preferred that the housing comprises a light source (like an LED) and/or a sound source (like a beeper) and/or a haptic source (like a buzzer), such signals (or sources) are arranged to give feedback to the caregiver upon attachment to the absorbent article to indicate the status for the device, i.e. if interconnection is properly made to the article and if connectivity for data transmission is appropriate and operational.

Preferably, when attached to the absorbent article and in the closed position, a light source, such as an LED light, is exposed above where the housing sinks into the pocket; or the at least one layer is translucent enough such that colour differences from the LED light can be seen through said layer. It is advantageous for the layer, such as the backsheet of the absorbent article to be interposed between the housing and the clip element to provide a secure fit, this arrangement allows for LED light's colors to be further differentiated by a caregiver so that colour information can be related to a proper placement of the device and/or device status.

In an embodiment, the absorbent article comprises a pocket (116) sized to accommodate the housing (2) of the detection device (1) therein and to expose at least a potion thereof, said portion corresponding substantially to the size of the display (9). Advantageously, the pocket is sized precisely to accommodate the detection devices herein so as to make it as intuitive and simple for the caregivers to assemble and manipulate.

In an embodiment, the absorbent article comprises a pocket (116) sized to accommodate the housing (2) of the detection device (1) therein typically wherein the slit (or opening) is sized such that the withdrawal end (13) of the housing is physically restricted from being inserted into the pocket. Preferably, the withdrawal end (13) comprises a perimeter that is greater than the perimeter of the slit (or opening) preferably at least 1.02, 1.05, 1.1, or 1.2, times greater, even more preferably from 1.08 to 1.3 times greater. Advantageously, the pocket is sized precisely to accommodate the detection devices herein so as to make it as intuitive and simple for the caregivers to assemble and manipulate and particularly the slit is sized precisely to avoid the clip-on from being inserted too deeply. Furthermore advantageously, this is a way to avoid the device from being inserted too deeply into the pocket that can be an alternative (or used in conjunction) with the use of an adhesive to create a seal barrier at the bottom side of said pocket.

In an embodiment, the pocket has a width Wp that is from A to B, wherein A is equal to (0.95*perimeter of the housing cross-section)/2 and B is equal to (1*perimeter of the housing cross-section)/2. Typically wherein the cross-section is taken about an axis crossing the y axis and substantially perpendicular thereto. Advantageously, once inserted into the pocket this prevents the housing from being shifted sideways out of its intended position or from being tilted out of its intended position.

In an embodiment, at least one layer of said absorbent article comprises a liquid impermeable backsheet of said absorbent article, preferably consists of a said liquid impermeable backsheet and a further liquid permeable nonwoven layer laminated to a garment facing surface of said backsheet. Advantageously, no additional layers or webs need to be added to the absorbent article in order to connect the detection device with the absorbent article.

In an embodiment, the clip element (6) is removably connected to the housing (2) typically such that it may be replaced with a new one in case of damage or excessive soiling. The clip element, which is externally exposed, is the portion of the detection device that is more prone to damages such as scratches, chips or soiling, advantageously this allows for such component to be easily and quickly removed and replaced when needed.

In a preferred embodiment, the clip element (6) is free of any electronics (such as electric wires, sensors, displays, battery, processors etc.). Advantageously, this renders the clip element easy and cheap to replace.

In an embodiment, the housing (2) is sealed such that it is resistant to liquid and is at least splash-proof and/or dust-proof, preferably has a Ingress Protection rating selected from the group consisting of IPn4, IPn5, or IPn6; wherein n is equal to X, 1, 2, 3, 4, 5, or 6, according to method ISO 20653:2013, preferably at least an Ingress Protection rating of at least IP65 or higher. Advantageously, the detection device is thus dust and/or liquid resistant so that exudates (e.g. urine) cannot permeate therethrough and damage the internal electronics within the housing.

In an embodiment, the clip element (6) comprises two or more locking elements (8,8') oppositely disposed on either side of a longitudinal centerline axis (y) running between said locking elements (8,8') and being parallel to a longest edge of said clip element (6), and wherein each said locking elements (8,8') are arranged to fasten onto the housing (2) by mechanically engaging with oppositely disposed side edges (5) of the housing (2). Preferably, the locking elements (8,8') are free of sharp edges and comprise one or more rounded protrusions. Advantageously this allows for a secure fit when one or more layers of the absorbent article are to be positioned between the housing and the clip element, whilst limiting the risk of tearing of said layers as well as secured conductive interconnection upon usage and mechanically secured against unintended removal.

In an embodiment, the housing (2) further comprises a push button (11) positioned on the front face (3) such that it may be accessed and pressed by a subject when the detection device (1) is in the open position and wherein when the detection device is in the closed position said button (11) is covered by the clip element (6) so that it cannot be accessed and/or pressed by a subject, and/or so that it cannot be pressed unintentionally. The button may be pressed by a caregiver for at least one of the following: confirming that a detection device was intentionally taken off an absorbent article (e.g. when pushed before a predefined time interval has elapsed, so that no "not-worn alarm" will be generated) for example by a short press (e.g. press for 1 second or less); to stop an alarm (e.g. if abovementioned not-worn alarm had been activated) for example by a short press (e.g. press for 1 second or less) after the alarm has started; unpairing the detection device from an absorbent article (e.g. removing patient link to the detection device without replacing with a new detection device for that patient) for example by a medium press (e.g. pressing for between 2 seconds and 5 seconds); resetting the detection device for example by a long press (e.g. press for 10 seconds to 15 seconds). This arrangement advantageously allows for the button to be protected when in use and be easily manipulated by a caregiver once the detection device has been removed from the absorbent article.

In an embodiment, the housing (2) has an insertion end (12) and a withdrawal end (13), and wherein the insertion end (12) has a width that is less than a width of said withdrawal end (13), wherein said width is taken along an axis perpendicular to the longitudinal centerline axis (y), and/or wherein said insertion end (12) is elongatedly shaped such that an apex is formed at a position of said insertion end (12) substantially intercepting said longitudinal centerline axis (y). It has been found that such shapes advantageously ease insertion of the detection device into the pocket and aid the caregivers to insert the detection device in the correct orientation and not the other way around.

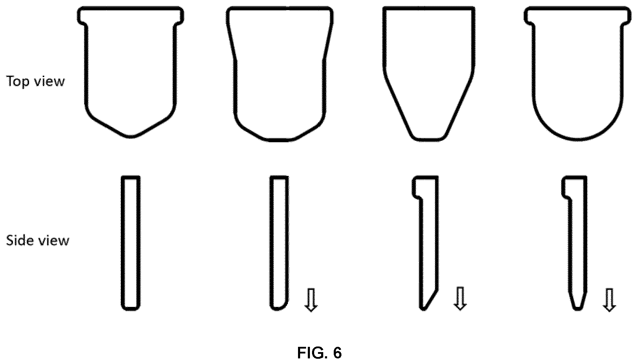

In an embodiment, as exemplified in Fig. 6, the housing (2) may have a number of suitable shapes provided that the insertion end has a smaller cross-sectional perimeter than the withdrawal end. Most preferably, the insertion end also has a thickness that is less than the thickness of the withdrawal end, for example the insertion end may be tapered about two or more different planes.

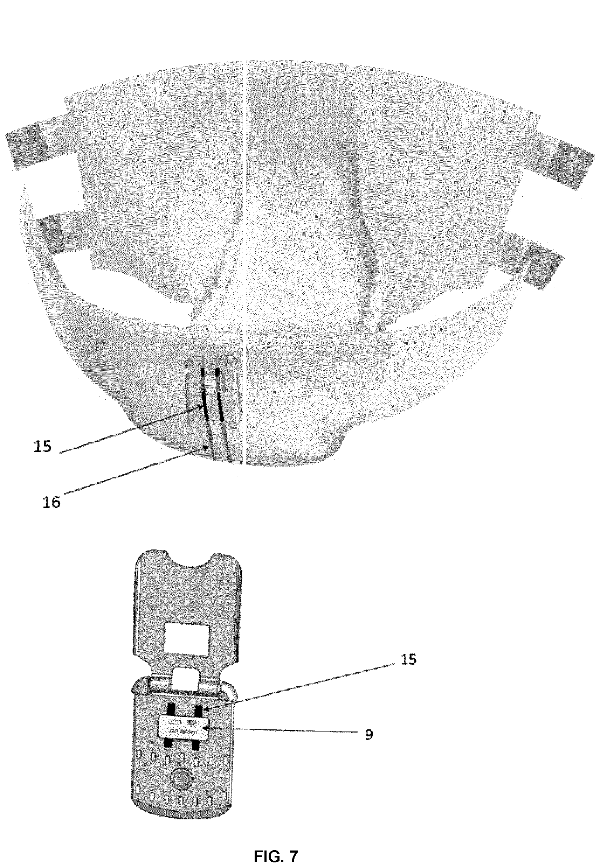

In a preferred embodiment, as exemplified in Fig. 7, the detection device (1) comprises first indicia (15) that is to be aligned with one or more second indicia (16) positioned on the backsheet of the absorbent article, typically such that a visual indication of correct alignment of the detection device (1) into the pocket of the absorbent article is achieved. Preferably, the second indicia (16) comprises, preferably consists of, at least a portion of the conductive sensor tracks (101) that are colored differently from the color of the backsheet. More preferably, the first indicia (15) comprises one or more marks (e.g. printed marks or stickers) onto the housing (such as above and/or below the display (9)); one or more marks (e.g. printed marks or stickers) on an outward surface of the clip element (such as proximal to the window or cut-out), and/or one or more marks (e.g. one or more bars) displayed on the display (9). Advantageously, this embodiment allows for the caregiver to very easily and intuitively ensure correct alignment of the terminals of the detection device with the conductive sensor tracks simply by ensuring visual alignment of the first and second indicia is attained.

In an embodiment, a kit is provided comprising a plurality of absorbent articles (100) as described herein, and from 1 to less than 5, preferably less than 4, more preferably less than 3, detection devices (1) as described herein.

In an embodiment, an array is provided comprising a plurality of absorbent articles (100) as described herein, wherein said array comprises absorbent articles having at least two different sizes and wherein a plurality of absorbent articles of at least one of the at least two different sizes further comprise an absorbent core comprising one or more channels substantially free of absorbent material, preferably wherein the absorbent core comprises one or more core wrap layers enclosing absorbent material therein and wherein a top layer of said core wrap is joined to a bottom layer of said core wrap to form said channels substantially free of absorbent material, preferably said channels being circumscribed by absorbent material. Preferably the array comprises more than two detection devices as described herein.

It is supposed that the present invention is not restricted to any form of realization described previously and that some modifications can be added to the presented example of fabrication without reappraisal of the appended claims.

## Claims

1. A detection device (1) comprising:
a housing (2) comprising a front face (3), a back face (4), and side edges (5) connecting the front and back faces (3,4) and disposed therebetween, wherein said housing (2) comprises therein a battery, a processor, and a transmitter; and
a clip element (6) rotatably connected to the housing (2),
wherein the clip element (6) comprises one or more locking elements (7,7') arrange to mechanically engage with at least a portion of the side edge (5) when in a closed position, and wherein said clip element (6) can be rotated from an open position, where the locking elements (7,7') do not mechanically engage with said side edge (5), to said closed position, and wherein said detection device (1) is suitable for coupling to an absorbent article (100) and arranged such that when in said closed position at least one layer of said absorbent article (100) is interposed between said housing (2) and said clip element (6); wherein said housing (2) further comprises a plurality of conductive terminals (8,8') that are exposed on the front face (3) of said housing (2) and facing said clip element (6) when in the closed position, said conductive terminals (8,8') being arranged to come into contact with one or more conductive sensor tracks (101) positioned on a surface of the absorbent article (100).

2. A detection device (1) according to Claim 1, wherein said surface, is a body facing surface, of the at least one layer of said absorbent article interposed between the housing (2) and the clip element (6) when in the closed position, said layer being a liquid impermeable backsheet of said absorbent article (100).

3. A detection device (1) according to any of the preceding Claims, wherein the conductive terminals (8,8') are more than 4, and preferably from 6 to 20, and wherein said conductive terminals (8,8') are arranged in two or more rows along a length of the front face (3) of the housing (2) typically extending parallel to a longitudinal axis (y).

4. A detection device (1) according to any of the preceding Claims, further comprising first indicia (15) arranged to provide a visual indication that said detection device is appropriately aligned with one or more conductive sensor tracks (101) positioned on the absorbent article (100), wherein said first indicia (15) comprises, one or more marks positioned on the housing; one or more marks on an outward surface of the clip element, and/or one or more marks displayed on the display (9).

5. A detection device (1) according to any of the preceding Claims, wherein the housing (2) comprises a display (9) adapted to display information relating to a monitoring status and/or detection device status, and wherein the clip element (6) comprises a cut-out (10) or window sized such that the display (9) may protrude or be seen therethrough when in the closed position, preferably such that when in the closed position the display (9) can be viewed by a caregiver.

6. A detection device (1) according to Claim 5, wherein, when attached to the absorbent article and in the closed position, a light source is exposed from the at least one layer of said absorbent article interposed between the housing (2) and the clip element (6); or the at least one layer is translucent such that said light source can be seen through said layer;

7. A detection device (1) according to any of the preceding Claims, wherein the absorbent article comprises a pocket (116) seized to accommodate the housing (2) of the detection device (1) therein and to expose at least a potion thereof, said portion corresponding at least to shoulders (14) of the housing (2).

8. A detection device (1) according to any of the preceding Claims, wherein at least one layer of said absorbent article comprises a liquid impermeable backsheet of said absorbent article, preferably consists of a said liquid impermeable backsheet and a further liquid permeable nonwoven layer laminated to a garment facing surface of said backsheet.

9. A detection device (1) according to any of the preceding Claims, wherein the clip element (6) is removably connected to the housing (2) such that it may be replaced with a new one in case of damage or excessive soiling.

10. A detection device (1) according to any of the preceding claims wherein the housing (2) is sealed such that it is resistant to liquid and is at least splash-proof.

11. A detection device (1) according to any of the preceding Claims, wherein the clip element (6) comprises two or more locking elements (8,8') oppositely disposed on either side of a longitudinal centerline axis (y) running between said locking elements (8,8') and being parallel to a longest edge of said clip element (6), and wherein each said locking elements (8,8') are arranged to fasten onto the housing (2) by mechanically engaging with oppositely disposed side edges (5) of the housing (2).

12. A detection device (1) according to any of the preceding Claims, wherein the housing (2) further comprises a push button (11) positioned on the front face (3) such that it may be accessed and pressed by a subject when the detection device (1) is in the open position and wherein when the detection device is in the closed position said button (11) is covered by the clip element (6) so that it cannot be accessed and/or pressed by a subject or accidentally pushed due to movements.

13. A detection device (1) according to any of the preceding Claims, wherein the housing (2) has an insertion end (12) and a withdrawal end (13), and wherein the insertion end (12) has a width that is less than a width of sa d withdrawal end (13), wherein said width is taken along an axis perpendicular to the longitudinal centerline axis (y), and/or wherein said insertion end (12) is elotigatedly shaped such that an apex is formed at a position of said insertion end (12) substantially intercepting said longitudinal centerline axis (y).

14. An absorbent article (100) comprising a detection device (1) according to any of the preceding Claims, removably attached thereto.

15. An absorbent article (100) according to Claim 14, comprising a liquid permeable topsheet, a liquid impermeable backsheet comprising an opening (115) for the detection device (1) to be inserted therein, and an absorbent core positioned between said topsheet and backsheet, wherein an insulating strip is positioned between said backseet and said absorbent core forming a pocket (116) adapted to retain the housing (2) of said detection device (1) therein.

16. An array comprising a plurality of absorbent articles (100) according to Claims 14 to 15, wherein said array comprises absorbent articles having at least two different sizes and wherein a plurality of absorbent articles of at least one of the at least two different sizes further comprise an absorbent core comprising one or more channels substantially free of absorbent material, wherein the absorbent core comprises one or more core wrap layers enclosing absorbent material therein and wherein a top layer of said core wrap is joined to a bottom layer of said core wrap to form said channels substantially free of absorbent material, preferably said channels being circumscribed by absorbent material.

## Patentansprüche

1. Detektionsvorrichtung (1), umfassend:
ein Gehäuse (2), umfassend eine Vorderseite (3), eine Rückseite (4) und Seitenkanten (5), die die Vorder- und Rückseite (3,4) verbinden und zwischen ihnen angeordnet sind, wobei das Gehäuse (2) eine Batterie, einen Prozessor und einen Sender umfasst; und
ein Klemmelement (6), das mit dem Gehäuse (2) drehbar verbunden ist,
wobei das Klemmelement (6) ein oder mehrere Verriegelungselemente (7,7') umfasst, die eingerichtet sind, um mit mindestens einem Abschnitt der Seitenkante (5) mechanisch in Eingriff zu stehen, wenn sie sich in einer geschlossenen Position befinden, und wobei das Klemmelement (6) aus einer offenen Position, in der die Verriegelungselemente (7,7') nicht mechanisch mit der Seitenkante (5) in Eingriff stehen, in die geschlossene Position gedreht werden kann und wobei die Detektionsvorrichtung (1) zum Koppeln mit einem Absorptionsartikel (100) geeignet ist und derart eingerichtet ist, das, wenn es sich in der geschlossenen Position befindet, mindestens eine Schicht des Absorptionsartikels (100) zwischen dem Gehäuse (2) und dem Klemmelement (6) eingefügt ist; wobei das Gehäuse (2) ferner eine Vielzahl von leitfähigen Anschlüssen (8,8') umfasst, die auf der Vorderseite (3) des Gehäuses (2) freiliegen und dem Klemmelement (6) zugewandt sind, wenn es sich in der geschlossenen Position befindet, die leitfähigen Anschlüsse (8,8') eingerichtet sind, um mit einer oder mehreren leitfähigen Sensorbahnen (101) in Kontakt zu treten, die auf einer Oberfläche des Absorptionsartikels (100) positioniert sind.

2. Detektionsvorrichtung (1) nach Anspruch 1, wobei die Oberfläche eine dem Körper zugewandte Oberfläche der mindestens einen Schicht des Absorptionsartikels ist, die zwischen dem Gehäuse (2) und dem Klemmelement (6) eingefügt ist, wenn es sich in der geschlossenen Position befindet, wobei die Schicht eine flüssigkeitsundurchlässige Rückschicht des Absorptionsartikel (100) ist.

3. Detektionsvorrichtung (1) nach einem der vorstehenden Ansprüche, wobei die leitfähigen Anschlüsse (8,8') mehr als 4 und vorzugsweise 6 bis 20 sind und wobei die leitfähigen Anschlüsse (8,8') in zwei oder mehr Reihen entlang einer Länge der Vorderseite (3) des Gehäuses (2) eingerichtet sind, die sich typischerweise parallel zu einer Längsachse (y) erstreckt.

4. Detektionsvorrichtung (1) nach einem der vorstehenden Ansprüche, ferner umfassend erste Zeichen (15), die eingerichtet sind, um eine visuelle Anzeige dafür bereitzustellen, dass die Detektionsvorrichtung richtig mit einer oder mehreren leitfähigen Sensorspuren (101) in Ausrichtung gebracht ist, die auf dem Absorptionsartikel (100) positioniert sind, wobei die ersten Zeichen (15) eine oder mehrere Markierungen umfassen, die auf dem Gehäuse positioniert sind; eine oder mehrere Markierungen auf einer Außenoberfläche des Klemmelements und/oder eine oder mehrere Markierungen, die auf dem Display (9) angezeigt werden.

5. Detektionsvorrichtung (1) nach einem der vorstehenden Ansprüche, wobei das Gehäuse (2) ein Display (9) umfasst, das geeignet ist, um Informationen in Bezug auf einen Überwachungsstatus und/oder Detektionsvorrichtungsstatus anzuzeigen, und wobei das Klemmelement (6) einen Ausschnitt (10) oder ein Fenster umfasst, der/das derart bemessen ist, dass das Display (9) hervorstehen oder dadurch hindurch gesehen werden kann, wenn es sich in der geschlossenen Position befindet, vorzugsweise derart, dass, wenn das Display (9) von einer Pflegekraft gesehen werden kann, wenn es sich in der geschlossenen Position befindet.

6. Detektionsvorrichtung (1) nach Anspruch 5, wobei, wenn sie an dem Absorptionsartikel angebracht ist und sich in der geschlossenen Position befindet, eine Lichtquelle von der mindestens einen Schicht des Absorptionsartikel frei liegt, die zwischen dem Gehäuse (2) und dem Klemmelement (6) eingefügt ist; oder die mindestens eine Schicht derart durchscheinend ist, dass die Lichtquelle durch die Schicht hindurch sichtbar ist.

7. Detektionsvorrichtung (1) nach einem der vorstehenden Ansprüche, wobei der Absorptionsartikel eine Tasche (116) umfasst, die bemessen ist, um das Gehäuse (2) der Detektionsvorrichtung (1) darin aufzunehmen und um mindestens einen Abschnitt davon freizugeben, wobei der Abschnitt mindestens Schultern (14) des Gehäuses (2) entspricht.

8. Detektionsvorrichtung (1) nach einem der vorstehenden Ansprüche, wobei mindestens eine Schicht des Absorptionsartikels eine flüssigkeitsundurchlässige Rückschicht des Absorptionsartikels umfasst, vorzugsweise aus der flüssigkeitsundurchlässigen Rückschicht und einer weiteren flüssigkeitsdurchlässigen Vliesschicht besteht, die auf eine dem Kleidungsstück zugewandte Oberfläche der Rückschicht laminiert ist.

9. Detektionsvorrichtung (1) nach einem der vorstehenden Ansprüche, wobei das Klemmelement (6) mit dem Gehäuse (2) derart lösbar verbunden ist, dass es im Falle einer Beschädigung oder übermäßigen Verschmutzung durch ein neues ersetzt werden kann.

10. Detektionsvorrichtung (1) nach einem der vorstehenden Ansprüche, wobei das Gehäuse (2) derart abgedichtet ist, dass es gegen Flüssigkeit beständig und mindestens spritzwassergeschützt ist.

11. Detektionsvorrichtung (1) nach einem der vorstehenden Ansprüche, wobei das Klemmelement (6) zwei oder mehr Verriegelungselemente (8,8') umfasst, die gegenüberliegend auf beiden Seiten einer Längsmittelachse (y) angeordnet sind, die zwischen den Verriegelungselementen (8,8') verläuft und parallel zu einer längsten Kante des Klemmelements (6) liegt, und wobei jedes der Verriegelungselemente (8,8') eingerichtet ist, um durch mechanisches Ineingriffnehmen in gegenüberliegend angeordnete Seitenkanten (5) des Gehäuses (2) auf dem Gehäuse (2) befestigt wird.

12. Detektionsvorrichtung (1) nach einem der vorstehenden Ansprüche, wobei das Gehäuse (2) ferner einen Druckknopf (11) umfasst, der auf der Vorderseite (3) derart positioniert ist, dass er durch ein Subjekt erreicht und gedrückt werden kann, wenn sich die Detektionsvorrichtung (1) in der geöffneten Position befindet, und wobei der Knopf (11), wenn sich die Detektionsvorrichtung in der geschlossenen Position befindet, durch das Klemmelement (6) abgedeckt ist, sodass er nicht durch ein Subjekt erreicht und/oder gedrückt oder aufgrund von Bewegungen versehentlich gedrückt werden kann.

13. Detektionsvorrichtung (1) nach einem der vorstehenden Ansprüche, wobei das Gehäuse (2) ein Einführende (12) und ein Entnahmeende (13) aufweist und wobei das Einführende (12) eine Breite aufweist, die geringer als eine Breite des Entnahmeendes (13) ist, wobei die Breite entlang einer Achse senkrecht zu der Längsmittelachse (y) gemessen wird und/oder wobei das Einführende (12) länglich derart geformt ist, dass an einer Position des Einführendes (12) eine Spitze ausgebildet wird, die die Längsmittelachse (y) im Wesentlichen schneidet.

14. Absorptionsartikel (100), umfassend eine Detektionsvorrichtung (1) nach einem der vorstehenden Ansprüche, die lösbar daran angebracht ist.

15. Absorptionsartikel (100) nach Anspruch 14, umfassend eine flüssigkeitsdurchlässige Oberschicht, eine flüssigkeitsundurchlässige Rückschicht, umfassend eine Öffnung (115) zum Einsetzen der Detektionsvorrichtung (1) darin, und einen Absorptionskern, der zwischen der Oberschicht und der Rückschicht positioniert ist, wobei zwischen der Rückschicht und dem Absorptionskern ein Isolierstreifen positioniert ist und der Absorptionskern eine Tasche (116) ausbildet, die geeignet ist, um das Gehäuse (2) der Detektionsvorrichtung (1) darin zu halten.

16. Anordnung, umfassend eine Vielzahl von Absorptionsartikeln (100) nach Anspruch 14 bis 15, wobei die Anordnung Absorptionsartikel umfasst, die mindestens zwei unterschiedlichen Größen aufweisen und wobei eine Vielzahl von Absorptionsartikeln mindestens einer der mindestens zwei unterschiedlichen Größen ferner einen Absorptionskern umfasst, umfassend einen oder mehrere Kanäle, die im Wesentlichen frei von Absorptionsmaterial sind, wobei der Absorptionskern eine oder mehrere Kernumhüllungsschichten umfasst, die Absorptionsmaterial darin umschließen, und wobei eine obere Schicht der Kernumhüllung mit einer unteren Schicht der Kernumhüllung verknüpft ist, um die Kanäle im Wesentlichen frei von Absorptionsmaterial auszubilden, vorzugsweise wobei die Kanäle von Absorptionsmaterial umgeben sind.

## Revendications

1. Dispositif de détection (1) comprenant :
un boîtier (2) comprenant une face avant (3), une face arrière (4) et des bords latéraux (5) reliant les faces avant et arrière (3, 4) et disposés entre elles, dans lequel ledit boîtier (2) comprend à l'intérieur de celui-ci une batterie, un processeur, et un émetteur ; et
un élément de fixation (6) relié de manière pivotante au boîtier (2),
dans lequel l'élément de fixation (6) comprend un ou plusieurs éléments de verrouillage (7, 7') agencés pour venir en prise mécaniquement avec au moins une partie du bord latéral (5) lorsqu'il est dans une position fermée, et dans lequel ledit élément de fixation (6) peut être pivoté depuis une position ouverte, où les éléments de verrouillage (7, 7') ne viennent pas en prise mécaniquement avec ledit bord latéral (5), jusqu'à ladite position fermée, et dans lequel ledit dispositif de détection (1) est approprié pour un accouplement à un article absorbant (100) et agencé de telle sorte que lorsqu'il est dans ladite position fermée au moins une couche dudit article absorbant (100) est intercalée entre ledit boîtier (2) et ledit élément de fixation (6) ; dans lequel ledit boîtier (2) comprend en outre une pluralité de bornes conductrices (8, 8') qui sont exposées sur la face avant (3) dudit boîtier (2) et faisant face vers l'élément de fixation (6) lorsqu'il est dans la position fermée, lesdites bornes conductrices (8, 8') étant agencées pour entrer en contact avec une ou plusieurs pistes de capteur conductrices (101) positionnées sur une surface de l'article absorbant (100).

2. Dispositif de détection (1) selon la revendication 1, dans lequel ladite surface, est une surface faisant face vers le corps, de l'au moins une couche dudit article absorbant interposée entre le boîtier (2) et l'élément de fixation (6) lorsqu'il est dans la position fermée, ladite couche étant une feuille arrière imperméable aux liquides dudit article absorbant (100).

3. Dispositif de détection (1) selon l'une quelconque des revendications précédentes, dans lequel les bornes conductrices (8, 8') sont de plus de 4, et de préférence de 6 à 20, et dans lequel lesdites bornes conductrices (8, 8') sont agencées en deux rangées ou plus le long d'une longueur de la face avant (3) du boîtier (2) s'étendant de manière générale parallèlement à un axe longitudinal (y).

4. Dispositif de détection (1) selon l'une quelconque des revendications précédentes, comprenant en outre un premier indice (15) agencé pour fournir une indication visuelle que ledit dispositif de détection est aligné de manière appropriée avec une ou plusieurs pistes de capteur conductrices (101) positionnées sur l'article absorbant (100), dans lequel ledit premier indice (15) comprend une ou plusieurs marques positionnées sur le boîtier ; une ou plusieurs marques sur une surface extérieure de l'élément de fixation, et/ou une ou plusieurs marques affichées sur l'écran (9).

5. Dispositif de détection (1) selon l'une quelconque des revendications précédentes, dans lequel le boîtier (2) comprend un écran (9) adapté pour afficher des informations relatives à un état de surveillance et/ou un état du dispositif de détection, et dans lequel l'élément de fixation (6) comprend une découpe (10) ou une fenêtre dimensionnée de telle sorte que l'écran (9) peut faire saillie ou être vu à travers lorsqu'il est dans la position fermée, de préférence de telle sorte que lorsqu'il est dans la position fermée l'écran (9) peut être vu par un soignant.

6. Dispositif de détection (1) selon la revendication 5, dans lequel, lorsqu'il est fixé à l'article absorbant et dans la position fermée, une source lumineuse est exposée depuis l'au moins une couche dudit article absorbant intercalée entre le boîtier (2) et l'élément de fixation (6) ; ou l'au moins une couche est translucide de telle sorte que ladite source lumineuse peut être vue à travers ladite couche.

7. Dispositif de détection (1) selon l'une quelconque des revendications précédentes, dans lequel l'article absorbant comprend une poche (116) dimensionnée pour accueillir le boîtier (2) du dispositif de détection (1) à l'intérieur de celle-ci et pour exposer au moins une partie de celui-ci, ladite partie correspondant au moins aux épaulements (14) du boîtier (2).

8. Dispositif de détection (1) selon l'une quelconque des revendications précédentes, dans lequel au moins une couche dudit article absorbant comprend une feuille arrière imperméable aux liquides dudit article absorbant, de préférence se compose d'une dite feuille arrière imperméable aux liquides et d'une autre couche non tissée perméable aux liquides stratifiée sur une surface faisant face vers le vêtement de ladite feuille arrière.

9. Dispositif de détection (1) selon l'une quelconque des revendications précédentes, dans lequel l'élément de fixation (6) est relié de manière amovible au boîtier (2) de telle sorte qu'il peut être remplacé par un nouveau en cas de dommage ou d'encrassement excessif.

10. Dispositif de détection (1) selon l'une quelconque des revendications précédentes, dans lequel le boîtier (2) est scellé de telle sorte qu'il est résistant aux liquides et est au moins étanche aux éclaboussures.

11. Dispositif de détection (1) selon l'une quelconque des revendications précédentes, dans lequel l'élément de fixation (6) comprend deux éléments de verrouillage (8, 8') ou plus disposés de manière opposée de part et d'autre d'un axe de ligne centrale longitudinal (y) passant entre lesdits éléments de verrouillage (8, 8') et étant parallèle à un bord le plus long dudit élément de fixation (6), et dans lequel chacun desdits éléments de verrouillage (8, 8') est agencé pour se fixer sur le boîtier (2) en venant en prise mécaniquement avec des bords latéraux (5) du boîtier (2) disposés de manière opposée.

12. Dispositif de détection (1) selon l'une quelconque des revendications précédentes, dans lequel le boîtier (2) comprend en outre un bouton poussoir (11) positionné sur la face avant (3) de telle sorte qu'il peut être accessible et pressé par un sujet lorsque le dispositif de détection (1) est dans la position ouverte et dans lequel lorsque le dispositif de détection est dans la position fermée ledit bouton (11) est recouvert par l'élément de fixation (6) de sorte qu'il ne puisse pas être accessible et/ou pressé par un sujet ou poussé accidentellement en raison de mouvements.

13. Dispositif de détection (1) selon l'une quelconque des revendications précédentes, dans lequel le boîtier (2) a une extrémité d'insertion (12) et une extrémité de retrait (13), et dans lequel l'extrémité d'insertion (12) a une largeur qui est inférieure à une largeur de ladite extrémité de retrait (13), dans lequel ladite largeur est prise le long d'un axe perpendiculaire à l'axe de ligne centrale longitudinal (y), et/ou dans lequel ladite extrémité d'insertion (12) est de profil allongé de telle sorte qu'un apex est formé au niveau d'une position de ladite extrémité d'insertion (12) interceptant sensiblement ledit axe de ligne centrale longitudinal (y).

14. Article absorbant (100) comprenant un dispositif de détection (1) selon l'une quelconque des revendications précédentes, attaché de manière amovible à celui-ci.

15. Article absorbant (100) selon la revendication 14, comprenant une feuille supérieure perméable aux liquides, une feuille arrière imperméable aux liquides comprenant une ouverture (115) pour le dispositif de détection (1) à insérer à l'intérieur de celle-ci, et un noyau absorbant positionné entre lesdites feuille supérieure et feuille arrière, dans lequel une bande isolante est positionnée entre ladite feuille arrière et ledit noyau absorbant formant une poche (116) adaptée pour retenir le boîtier (2) dudit dispositif de détection (1) à l'intérieur de celle-ci.

16. Réseau comprenant une pluralité d'articles absorbants (100) selon les revendications 14 à 15, dans lequel ledit réseau comprend des articles absorbants ayant au moins deux tailles différentes et dans lequel une pluralité d'articles absorbants d'au moins l'une des au moins deux tailles différentes comprennent en outre un noyau absorbant comprenant un ou plusieurs canaux sensiblement exempts de matériau absorbant, dans lequel le noyau absorbant comprend une ou plusieurs couches d'enveloppe de noyau renfermant un matériau absorbant à l'intérieur de celles-ci et dans lequel une couche supérieure de ladite enveloppe de noyau est reliée à une couche inférieure de ladite enveloppe de noyau pour former lesdits canaux sensiblement exempts de matériau absorbant, de préférence lesdits canaux étant circonscrits par un matériau absorbant.
